(19) Europäisches Patentamt
European Patent Office
Office européen des brevets

(11) Veröffentlichungsnummer: **0 317 884 B1**

(12) **EUROPÄISCHE PATENTSCHRIFT**

(45) Veröffentlichungstag der Patentschrift: **10.03.93**

(51) Int. Cl.$^5$: **C07C 65/24**

(21) Anmeldenummer: **88119071.4**

(22) Anmeldetag: **17.11.88**

Teilanmeldung 92100514.6 eingereicht am 17/11/88.

# (54) Teilfluorierte Carbonsäuren sowie Derivate davon, Verfahren zu ihrer Herstellung und Verwendung.

(30) Priorität: **24.11.87 DE 3739796**
**24.11.87 DE 3739797**
**24.11.87 DE 3739800**

(43) Veröffentlichungstag der Anmeldung:
**31.05.89 Patentblatt 89/22**

(45) Bekanntmachung des Hinweises auf die Patenterteilung:
**10.03.93 Patentblatt 93/10**

(84) Benannte Vertragsstaaten:
**BE CH DE FR GB IT LI NL**

(56) Entgegenhaltungen:
**EP-A- 0 034 413**
**US-A- 4 196 277**
**US-A- 4 650 850**

(73) Patentinhaber: **HOECHST AKTIENGESELLSCHAFT**
**Postfach 80 03 20**
**W-6230 Frankfurt am Main 80(DE)**

(72) Erfinder: **Röhrscheid, Freimund, Dr.**
**Amselweg 24**
**W-6233 Kelkheim (Taunus)(DE)**
Erfinder: **Siegemund, Günter, Dr.**
**Frankfurter Strasse 21**
**W-6238 Hofheim am Taunus(DE)**
Erfinder: **Lau, Jürgen, Dr.**
**Thüringer Weg 2**
**W-6238 Hofheim am Taunus(DE)**

EP 0 317 884 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf teilfluorierte Carbonsäuren, speziell auf eine teilfluorierte Dicarbonsäure sowie deren Säurechlorid und auf eine teilfluorierte Tetracarbonsäure und ihrem Anhydrid, Verfahren zu ihrer Herstellung und ihre Verwendung.

Die Oxidation von 2,2-Bis(4-methylphenyl)hexafluorpropan mit Luftsauerstoff in essigsaurem Medium in Gegenwart eines Katalysators aus Kobalt- und Bromionen ist bekannt (SU-644 777 = CA 90:P 168310 K (1979). Die Übertragung dieser Reaktion auf fluorhaltige Verbindungen, die zwei Hexafluorisopropylidenbrücken und zusätzlich eine Diphenyletherbrücke tragen, lag jedoch nicht nahe, da die Etherbrücke im allgemeinen das Reaktionsverhalten bei Oxidationsreaktionen verändert. Auch die Übertragung dieser Reaktion auf fluorhaltige Verbindungen, die eine 3,4-Dimethylphenylkonfiguration aufweisen, scheiterte aber, da die Ausbeute und Reinheit der erhaltenen Verbindungen nicht befriedigend war.

Ferner ist ein Verfahren zur Herstellung von Benzoltetracarbonsäuren und ihren Anhydriden durch Oxidation von tetraalkyl-substituierten Benzolen mit Sauerstoff oder freien Sauerstoff enthaltenden Gasen in Gegenwart von Schwermetallverbindungen und Bromverbindungen bei erhöhten Temperaturen bekannt (DE-A 21 12 009). Aus der Veröffentlichung geht hervor, daß die vollständige Oxidation von zwei nachbarständigen Methylgruppen erhebliche Probleme bereitet.

Nach Beendigung der Oxidationsreaktion finden sich im Reaktionsprodukt nicht oxidierte Methylgruppen, CHO- und $CH_2OH$-Gruppen. Die $CH_2OH$-Gruppe bildet mit der benachbarten Carboxylgruppe Phthalidringe.

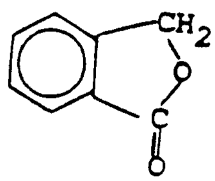

Zusätzlich erschwerend tritt hinzu, daß benachbarte Carboxygruppen mit Schwermetallen stabile Verbindungen bilden und als Metallsalze ausfallen können. Dieses setzt die katalytische Aktivität des Katalysators stark herab. Diese Metallsalzbildung wird sowohl bei der Phthalsäure als auch bei der Pyromellithsäure beobachtet.

Zur Verhinderung des Absinkens der katalytischen Aktivität des Katalysators und zur Unterstützung der Oxidation insbesondere einer vierten Alkylgruppe sei es erforderlich, daß bei der Reaktion große Mengen an Bromionen anwesend sein müssen.

Eine Übertragung dieses Verfahrens auf fluorhaltige Verbindungen ist aber nicht möglich, da durch Bromierungsreaktionen unter den angegebenen Versuchsbedingungen Produkte mit hohem Bromgehalt erhalten werden, die schwer abtrennbar sind und das Endprodukt nicht in genügender Reinheit und Ausbeute erhalten lassen. Darüber hinaus ist festgestellt worden, daß die teilfluorierten Polycarbonsäuren aus der Reaktionslösung gar nicht oder nur unvollständig auskristallisieren und zudem teilweise mit ihren Schwermetallsalzen vermischt sind, die eine Reinigung erheblich erschweren.

Dieses Verhalten ist um so überraschender, weil Polycarbonsäuren wie Terephthalsäure, Phthalsäure oder Pyromellithsäure sofort und in gut kristallisierter Form aus dem Reaktionsmedium wie Essigsäure auskristallisieren.

Teilfluorierte Tetracarbonsäuren mit 12 Fluoratomen (12F-Tetracarbonsäure) sind bisher nicht bekannt.

Es bestand daher die Aufgabe, ein Verfahren für teilfluorierte Carbonsäuren zu schaffen, das hohe Ausbeuten und eine hohe Reinheit der erhaltenen Produkte ermöglicht. So sind die teilfluorierten Tetracarbonsäuren und ihre Dianhydride beispielsweise Bausteine für Polyimide, die für technisch anspruchsvolle Zwecke verwendet werden, z.B. für thermisch hochbelastbare Überzüge und Klebstoffe im Flugzeugbau oder in der Mikroelektronik. Für viele dieser Anwendungsgebiete ist es daher wünschenswert, eine hohe Reinheit der eingesetzten Substanzen, beispielsweise von 99 % oder mehr zu fordern. Die Verwendung der nach dem erfindungsgemäßen Verfahren hergestellten Verbindungen in der Mikroelektronik macht es außerdem erforderlich, daß alle Metallionen, die aus dem Katalysator stammen oder während der Reaktionsschritte eingeschleppt werden, bis auf Konzentrationen im ppm-Bereich entfernt werden.

Die hochselektive Oxidation soll in der möglichst vollständigen Oxidation aller Methylgruppen der Methylverbindungen und in der Verhinderung von Nebenreaktionen wie Decarboxylierung und Kondensation bestehen. Um dieses Ziel zu erreichen, müssen spezielle Oxidationsbedingungen gefunden werden.

Gegenstand der Erfindung sind Verbindungen der Formel

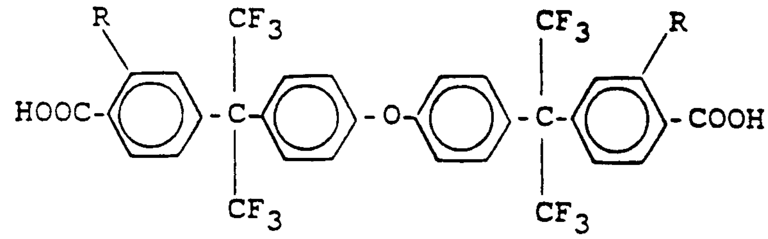

(I)

in der R gleich ist und Wasserstoff oder -COOH darstellt, sowie das Säurechlorid mit der Maßgabe, daß R Wasserstoff ist, und das Dianhydrid mit der Maßgabe, daß R den Rest -COOH darstellt.

Gegenstand der Erfindung ist auch ein Verfahren zur Herstellung

a) einer Verbindung der Formel

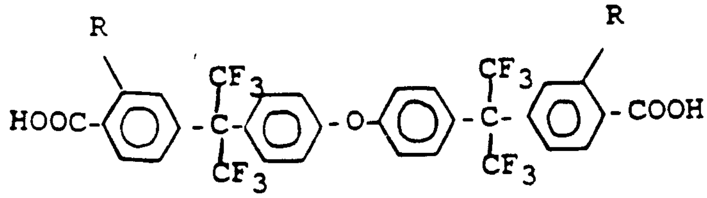

(II)

in der R gleich ist und Wasserstoff oder -COOH darstellt, oder

b) deren Anhydrid, wenn R nicht Wasserstoff ist, oder

c) das Säurechlorid, wenn R Wasserstoff ist,

durch Luftoxidation in saurem Medium bei erhöhtem Druck und erhöhter Temperatur in Gegenwart eines Katalysatorgemischs, dadurch gekennzeichnet, daß die entsprechende Methylverbindung in einem sauren organischen Medium durch Einleiten von Luftsauerstoff bei Temperaturen von 120 bis 220 °C und einem Druck zwischen 5 und 40 bar in Gegenwart von mindestens zwei Schwermetallverbindungen sowie von Bromidionen oxidiert und als solche isoliert wird oder das erhaltene Reaktionsgut in das Dianhydrid b) oder in das Säurechlorid c) der Verbindung der Formel (II) übergeführt wird.

Die Aufgabe der Erfindung wurde gelöst durch Einhalten besonderer Bedingungen für die Oxidation von den Methylverbindungen. Bei den entsprechenden Tetracarbonsäuren wurde eine Umsatzrate von über 90 % erreicht.

Folgende Maßnahmen haben sich dabei als günstig erwiesen:

1) Katalysator:

a) Wahl der Bestandteile für den Katalysator, insbesondere $Co^{2+}$, $Mn^{2+}$, $Ce^{3+}$, $Br^-$

b) optimales Verhältnis der Metallionen zueinander

c) hohe Gesamtmetallkonzentration in der Reaktionlösung

d) hohes Konzentrationsverhältnis von Metallionen : Bromidionen

2) Reaktionsbedingungen:

a) hoher Sauerstoffpartialdruck

b) kontrollierter Wassergehalt

Die Oxidation findet in einem sauren organischen Medium statt, bei der die Methylphenylverbindungen mit molekularem Sauerstoff oxidiert werden, wobei das saure Medium zu mindestens 40 % aus einer Monocarbonsäure wie Essigsäure oder Propionsäure oder deren Mischungen besteht. Essigsäure ist wegen ihrer größeren Beständigkeit gegen einen oxidativen Abbau vorzuziehen. Das Verhältnis von saurem Medium zu eingesetzter Ausgangssubstanz kann bis zu einem Verhältnis von 40 : 60 Gew.-%, bezogen auf die Gesamtreaktionsmasse, angewandt werden.

Die zur Herstellung der Di- bzw. Tetracarbonsäuren mit dem 12 F-Rest eingesetzten Diphenylether 4,4'-Bis[2-(4-methylphenyl)hexafluorisopropyl]diphenylether und 4,4'-Bis [2-(3,4-dimethylphenyl)-hexafluorisopropyl]diphenylether sind nach einem Verfahren erhältlich, das in der am selben Tage eingereichten europäischen Patentanmeldung, 88119070.6 die der deutschen Patentanmeldung P 37 39 795.8 entspricht, Titel "Teilfluorierte Diphenylether, Verfahren zu ihrer Herstellung und ihre Verwendung" beschrieben worden ist.

Das Katalysatorgemisch besteht aus mindestens zwei Schwermetallsalzen sowie Bromidionen. Als Schwermetalle werden beispielsweise Kobalt, Mangan oder Cer eingesetzt, wobei die Anwesenheit von Kobalt stets erforderlich ist. Bei der Herstellung der Dicarbonsäure wird z.B. ein Gemisch von Kobalt- und Manganionen eingesetzt, das zusätzlich Cerionen enthalten kann. Durch die Mischung der Metallsalze kann die Gesamtmetallkonzentration geringer gehalten werden, als wenn nur Kobalt allein eingesetzt wird.

Bromidionen sind für den vollständigen Ablauf der Oxidation unbedingt notwendig. Wird als Metallkomponente ein Gemisch von Kobalt- und Manganionen verwendet, so werden die Metalle im allgemeinen im Mol-Verhältnis von 3 : 1 bis 1 : 3, vorzugsweise 1 : 1 eingesetzt. Die Summe der Konzentrationen der beiden Elemente beträgt im allgemeinen 0,01 bis 0,2, vorzugsweise 0,02 bis 0,12 und insbesondere 0,04 bis 0,08 Grammatom/kg Gesamtmasse. Das Mol-Verhältnis der Summe von Kobalt und Mangan zu Brom ist im allgemeinen 1 : (0,01 bis 0,8), vorzugsweise 1 : (0,05 bis 0,4). Bei der Herstellung der Dicarbonsäure beträgt es im allgemeinen 1 : (0,01 bis 2), vorzugsweise 1 : (0,025 bis 1) und insbesondere 1 : (0,05 bis 0,2). Wie bereits erwähnt, ist es möglich, zusätzlich zu den beiden Metallionen des Katalysators auch Cerionen einzusetzen. Diese katalysieren die Oxidation unvollständig oxidierter Zwischenstufen. Ihre Anwesenheit erhöht die Reinheit und die Ausbeute der teilfluorierten Carbonsäuren. Die Cerionen werden dem Katalysator im Mol-Verhältnis der Summe der Kobalt- und Manganionen zu Cerionen wie 1 : (0,02 bis 1,2), vorzugsweise 1 : (0,05 bis 0,6) zugefügt. Bei den Dicarbonsäuren beträgt das Verhältnis 1 : (0,02 bis 2), vorzugsweise 1 : (0,05 bis 1) und insbesondere 1 : (0,2 bis 0,6). Wird ein Gemisch der Metallionen von Kobalt und Cer verwendet, so beträgt das Molverhältnis der beiden Metalle im allgemeinen 1 : (0,02 bis 1,2), wobei sich das Verhältnis der Metalle zu Brom wie vorstehend beschrieben ausweist. Die Molverhältnisse beziehen sich stets auf die Gesamtmasse, d.h. aus der Summe der zu oxidierenden Verbindung, Lösungsmittel und Katalysator. Vorzugsweise werden die Metallionen in Form ihrer Acetate eingesetzt.

Brom kann in Form von Bromiden, z.B. der Bromide der Alkalimetalle, einschließlich dem Ammoniumbromid, und denen der Metalle Kobalt, Mangan und Cer oder als Lösung von Bromwasserstoff in Wasser oder Eisessig eingesetzt werden. Daneben können auch bromhaltige organische Verbindungen verwendet werden, die während der Oxidation zerfallen und Bromionen freisetzen, z.B. Tetrabrommethan. Man kann die Bromidionen-Konzentration in der Reaktionslösung sehr stark - bis zu einem Wert von ca. 20 für das Molverhältnis $\Sigma M^n$:Br (Summe der Metallionen zu Bromionen) - senken, ohne daß es zu einer merklichen Abnahme der Reaktionsgeschwindigkeit kommt. Durch diese Maßnahme, die gleichzeitig auch das Verhältnis der Tetramethylverbindungen : Br in der Reaktionslösung erhöht, wird die unerwünschte Kernbromierung stark verringert.

Die Oxidation wird im allgemeinen bei Temperaturen von 120 bis 220, vorzugsweise 140 bis 190 und insbesondere 155 bis 180 °C durchgeführt. Der Druck im Reaktor liegt im allgemeinen zwischen 5 und 40, vorzugsweise zwischen 10 und 30 und insbesondere zwischen 14 bis 20 bar.

Für die Verfahrensweise ist es günstig, daß die zur Oxidation benötigte Luft nahe dem Boden des Reaktors in die Flüssigphase eingeleitet und durch starkes Rühren oder durch spezielle Düsen in der Flüssigphase fein verteilt wird. Besonders günstig ist es, ein Oxidationsgemisch einzufahren, dessen Sauerstoffgehalt durch Beimischen von reinem Sauerstoff auf einen Anteil von über 21 Volumen-% erhöht wurde. Durch diese Maßnahme werden hohe Sauerstoff-Partialdrucke in den in die Flüssigphase eintretenden Gasblasen erreicht. Es ist vorteilhaft, wenn der Sauerstoff-Partialdruck an der Austrittsstelle der Einleitungsvorrichtung mindestens 1 bar beträgt, vorzugsweise bei 2 bis 15 und insbesondere bei 3 bis 10 bar liegt.

Es ist für die Verfahrensführung weiterhin günstig, daß der Restsauerstoffgehalt des Abgases bestimmte Werte nicht unterschreitet. Der Sauerstoff-Partialdruck wird definiert durch die Formel

$$P_{O_2} = \text{Volumen-\% } O_2 \cdot (P_{ges} - P_{ac\text{-}Dampfdruck})$$

d.h. er ist das mathematische Produkt aus dem Restsauerstoffgehalt und der Differenz aus dem Gesamtdruck und dem Essigsäuredampfdruck (ac-Dampfdruck) bei der vorliegenden Reaktionstemperatur. Dieser Sauerstoff-Partialdruck in der Gasphase über der Reaktionslösung soll 0,2 bar nicht unterschreiten und liegt bevorzugt zwischen 0,35 und 2,8, insbesondere zwischen 0,45 und 1,3 bar.

Nach Beendigung der stark exothermen Reaktion ist es zweckmäßig, zur Vervollständigung der Oxidation aller Methylgruppen den Reaktor für 1 bis 3 Stunden, vorzugsweise für 2 Stunden, auf 150 bis 190 °C, vorzugsweise 160 bis 180 °C, bei einem Sauerstoffpartialdruck von 0,4 bis 2 bar, vorzugsweise 0,5 bis 1,3 bar zu halten.

Einen wesentlichen Einfluß auf die Durchführung des erfindungsgemäßen Verfahrens hat die Wasserkonzentration des sauren Mediums, in dem die Reaktion durchgeführt wird. Zwar können die Tetramethylverbindungen auch in - beispielsweise - Essigsäure mit einer Wasserkonzentration von 15 % und mehr oxidiert werden, jedoch vermindert sich dabei Ausbeute und vor allem die Reinheit der erhaltenen Produkte, und die Oxidation aller vier Methylgruppen läuft nur noch unvollständig ab. Andererseits wurde festgestellt, daß in wasserfreier Essigsäure die Metallionen des Katalysators durch die Tetracarbonsäuren ausgefällt und damit inaktiviert werden. Der Bereich für die Wasserkonzentration, in dem die Metallionen gelöst bleiben und in dem die Oxidation genügend vollständig abläuft, liegt zwischen 2 und 12, vorzugsweise zwischen 2 und 7 und insbesondere zwischen 3 und 5 % Wasser in der Monocarbonsäure z.B. Essigsäure.

Es wurde gefunden, daß überraschenderweise die Dianhydride der Tetracarbonsäuren in Eisessig oder Mischungen aus Eisessig und Acetanhydrid wenig löslich sind, wenn man durch geeignete Methoden das Wasser aus der Reaktionslösung entfernt und die Tetracarbonsäuren in ihre Anhydride überführt. Diese Umwandlung kann durch Destillation und/oder durch Zugabe von Essigsäureanhydrid geschehen. Die Dianhydride kristallisieren in hoher Ausbeute und gut filtrierbarer Form aus und wurden durch Waschen mit Eisessig, vorzugsweise einer Mischung aus Eisessig und Acetanhydrid, von den Metallsalzen und den löslichen Nebenprodukten befreit. Das Waschen mit einer Mischung aus Eisessig und Acetanhydrid ist besonders geeignet, da diese Mischung das Verbacken des Filterkuchens verhindert. Die Dianhydride fallen mit einer Reinheit von 94 bis 97 % an.

Überraschenderweise lösen sich auch die Metallsalze während der Anhydridbildung auf, so daß schon nach dieser ersten Reinigungsoperation der Gehalt aller Metallionen bei 50 bis 100 ppm liegt.

Die Umwandlung der Tetracarbonsäuren führt man vorzugsweise so aus, daß man aus der Reaktionslösung ein Gemisch von Essigsäure und Wasser abdestilliert und anschließend in der Hitze Acetanhydrid in einem kleinen Überschuß über die berechnete Menge Acetanhydrid zufügt (ca. 3 bis 12 % Acetanhydrid in der Lösung).

Eine besondere Verfahrensvariante besteht darin, daß bei erhöhter Temperatur und unter Druck von der Reaktionslösung über eine Kolonne Wasser abdestilliert wird. Unter diesen Bedingungen bilden sich ebenfalls die Dianhydride der Tetracarbonsäuren unter Wasserabspaltung. Gleichzeitig werden auch die Metallsalze wieder gelöst. Zur Vervollständigung der Umsetzung zum Dianhydrid wird am Ende soviel Acetanhydrid zugefügt, daß sich ca. 3 bis 12 % Acetanhydrid in der Lösung befinden.

Die Bildung von Dianhydrid durch destillative Entfernung von Wasser wird vorzugsweise bei einer Temperatur oberhalb 140 °C, nötigenfalls unter dem zusätzlichen Druck eines Inertgases durchgeführt. Die Essigsäure kann auch ganz oder teilweise durch eine andere aliphatische Carbonsäure wie Propionsäure, Hexansäure oder 2-Ethylhexansäure ersetzt werden.

Im allgemeinen werden die filtrierten und gewaschenen Dianhydride im Luftstrom, vorzugsweise unter vermindertem Druck, bei erhöhter Temperatur getrocknet.

Bei der Isolierung als Tetracarbonsäure ist es zweckmäßig, in Gegenwart einer anorganischen oder organischen Säure z.B. Salzsäure, zu arbeiten. Die Tetracarbonsäuren können vorteilhaft in einer gut filtrierbaren Form aus Wasser auskristallisiert werden, wenn geringe Konzentrationen von Essigsäure darin gelöst sind, vorzugsweise 6 bis 12 Gew.-% Essigsäure. Im allgemeinen wird so dabei verfahren, daß nach beendeter Oxidation aus der Reaktionslösung soviel Essigsäure abdestilliert wird, bis die Sumpftemperatur ca. 130 bis 155 °C beträgt und die Schmelze noch gut rührbar ist. In die heiße Schmelze wird heißes Wasser und gegebenenfalls Säure zugesetzt und die Lösung noch einmal, vorzugsweise unter Druck bis zu 2 Stunden auf 130 bis 150 °C erhitzt.

Die Tetracarbonsäuren kristallisieren als Hydrat aus und können durch vorsichtiges Trocknen bei Raumtemperatur als Hexahydrat isoliert werden. Durch Erhitzen auf 50 bis 80 °C im Gasstrom werden sie in die Tetracarbonsäuren und durch Erhitzen auf 180 bis 190 °C unter vermindertem Druck in die Dianhydride umgewandelt.

Besonders reine Produkte werden erhalten, wenn die noch wasserfeuchte Tetracarbonsäure in einem Lösungsmittel suspendiert und das Wasser abdestilliert wird. Dabei bildet sich zuerst die hydratfreie Tetracarbonsäure, aus der bei weiterer Temperaturerhöhung das Dianhydrid entsteht.

Besonders geeignet sind aromatische Lösemittel wie Toluol, o-Xylol, Tetrahydronaphthalin, Acetophenon oder Diphenylether.

Die Anhydridbildung in aromatischen Lösemitteln kann durch Zugabe katalytischer Mengen an Carbonsäuren, z.B. aliphatischen Carbonsäuren wie Essigsäure, 2-Ethylhexansäure, oder anderer Säuren wie Toluolsulfonsäure erheblich beschleunigt werden.

Zur Überführung in das Säurechlorid wird die nach dem erfindungsgemäßen Verfahren erhaltene Dicarbonsäure in bekannter Weise, z.B. mit Thionylchlorid behandelt und nach bekannten Methoden aus der Reaktionslösung gewonnen. Die Dicarbonsäure und ihr Säurechlorid können zur Herstellung von

linearen Polycarbonsäureamiden und -carbonsäureestern verwendet werden, die als Formkörper, Filme und Fasern hohe thermische Stabilität, ausgezeichnete mechanische Eigenschaften, gute Transparenz, gute schutzabweisende Eigenschaften und Strahlenbeständigkeit aufweisen.

Die Tetracarbonsäuren können zur Herstellung von Polykondensaten wie Polyimiden, Polycarbonsäureamiden, Polyamidcarbonsäureestern, Polyamiden und Imidoligomeren eingesetzt werden, die u.a. niedrige Schmelzpunkte, hohe Löslichkeit, niedrige Dielektrizitätskonstanten, erhöhte thermische Stabilität aufweisen.

In den nachfolgenden Beispielen sind % stets Gewichtsprozent.

**Beispiele**

1) 4,4'-Bis[2-(carboxyphenyl)hexafluorisopropyl]diphenylether(12-F-Dicarbonsäure)

250 g 4,4'-Bis[2-(4-methylphenyl)hexafluorisopropyl]diphenylether, 2,49 g $Co(OAc)_2 \cdot 4H_2O$, 2,45 g $Mn(OAc)_2 \cdot 4H_2O$, 0,41 g HBr = 4,1 g einer 10 %igen HBr-Lösung in Eisessig und 550 g Eisessig wurden im 1 l-Glasautoklav, der mit Rührer, Gaseinleitrohr, Thermometer und Rückflußkühler ausgerüstet war, vorgelegt. Unter 7,5 bar Sauerstoffdruck wurde bis auf maximal 180 °C erhitzt. Die exotherme Reaktion setzte unter Sauerstoffaufnahme bei ca. 130 °C ein und dauerte 40 Minuten. Die Endtemperatur von 175 °C wurde noch eine Stunde gehalten. Aus der auf ca. 100 °C abgekühlten Reaktionslösung wurden 300 g Essigsäure abdestilliert und der Destillationsrückstand unter Rühren auf 20 °C abgekühlt. Die gebildete Kristallsuspension wurde über eine Nutsche abgesaugt. Der Filterkuchen wurde mit vier Portionen zu 15 ml Eisessig und anschließend mit fünf Portionen zu 40 ml Wasser gewaschen. Das feuchte Produkt wurde bei 70 °C/65 mbar im leichten Luftstrom getrocknet. Ausbeute: 211,8 g (77,6 % d. Th.), Fp: 238-240 °C, farblose Kristalle, Carboxylgruppengehalt: 2,84 mVal COOH/g (ber. 2,82). Aus der Mutterlauge fiel durch Zugabe des Waschwassers zusätzliches Produkt aus. 57,3 g (21,0 % d. Th.), Fp: 227-232 °C.

| Analyse: $C_{32}H_{18}F_{12}O_5$ | | | |
|---|---|---|---|
| ber.: | C 54,08 % | H 2,53 % | F 32,11 % |
| gef.: | C 54,00 % | H 2,60 % | F 32,00 % |

2) 4,4'-Bis[2-(4-chlorcarbonylphenyl)hexafluorisopropyl]diphenylether (12-F-Dicarbonsäurechlorid)

Zu einer Aufschlämmung von 4,4'-Bis[2-(4-carboxyphenyl)hexafluorisopropyl]diphenylether in Thionylchlorid wurden einige Tropfen Dimethylformamid hinzugegeben und die Mischung unter Rückflußbedingungen erhitzt, bis die Entwicklung von Chlorwasserstoff beendet war. Der Überschuß an Thionylchlorid wurde abgezogen und Toluol zugegeben, um das restliche Thionylchlorid durch Destillation abzutrennen. Nachdem das Toluol entfernt worden war, wurde das Rohprodukt aus n-Hexan umkristallisiert. Fp.: 144-145 °C.

3) a) 4,4'-Bis[2-(3,4-dicarboxyphenyl)hexafluorisopropyl]diphenylether (12F-Tetracarbonsäure)

200,3 g 4,4'-Bis[2-(3,4-dimethylphenyl)hexafluorisopropyl]diphenylether, 2,49 g $Co(OAc)_2 \cdot 4\ H_2O$, 2,45 g $Mn(OAc)_2 \cdot 4\ H_2O$, 0,41 g HBr entsprechend 4,1 g 10 %iger HBr-Lösung in Eisessig und 550 g Eisessig wurden im 1 l-Glasautoklav, der mit Rührer, Gaseinleitrohr, Thermometer und Rückflußkühler ausgerüstet war, vorgelegt. Unter 7,5 bar Sauerstoffdruck wurde bis auf maximal 188 °C erhitzt. Die exotherme Reaktion setzte unter Sauerstoffaufnahme zwischen 90 und 100 °C ein und dauerte 65 Minuten. Die Endtemperatur von 177 °C wurde noch 1 1/4 Stunden gehalten. Es wurden 816 g Lösung isoliert.

b) Die auf 90 °C abgekühlte Reaktionslösung wurde filtriert und in einen 2 l-Vierhalskolben mit Rührer überführt, aus dem solange Essigsäure abdestilliert wurde, bis die Sumpftemperatur 140 °C betrug. Zu der violetten Schmelze wurde 1 l Wasser von 95 °C gegeben, wodurch sich eine Emulsion bildete. Unter schnellem Rühren wurden bei 80 °C 200 ml konz. Salzsäure zugefügt. Die Emulsion wurde bei 75 °C mit Kristallen der 12 F-Tetracarbonsäure angeimpft, wodurch sich beim Abkühlen eine Kristallsuspension bildete.

Die Kristallsuspension wurde bei 22 °C über eine Nutsche abgesaugt und zweimal mit je 200 ml 2n-Salzsäure und einmal mit 200 ml Wasser gewaschen und die wasserfeuchte Substanz bei 60 °C/65 mbar im leichten Luftstrom getrocknet.

Ausbeute: 227,2 g (96,4 %) 12 F-Tetracarbonsäure, farblose Kristalle, Fp. 163 bis 165 °C unter Wasserabspaltung, Carboxylgruppengehalt: 5,06 mVal COOH/g (ber. 5,01).

| Analyse für $C_{34}H_{18}F_{12}O_9$: | | | |
|---|---|---|---|
| ber.: | C 51,13 % | H 2,26 % | F 28,57 % |
| gef.: | C 51,00 % | H 2,20 % | F 28,35 % |

4) 12F-Tetracarbonsäuredianhydrid

Zur Reaktionslösung aus Beispiel 34a) wurde bei 95 °C die Lösung von 3,02 g Oxalsäuredihydrat in 30 ml Essigsäure unter Rühren zugetropft. Nach zwei Stunden am Rückfluß wurde die 100 °C heiße Lösung filtriert und das Filtrat wurde mit 200 ml heißer Essigsäure gewaschen. Vom Filtrat wurden 590 g Essigsäure und Wasser abdestilliert. Zum Rückstand wurden unter schnellem Rühren oberhalb 80 °C im Laufe von 30 Min. 72,3 g Acetanhydrid getropft. Die Temperatur stieg auf 120 °C und wurde für eine Stunde bei diesem Wert gehalten (12 % Acetanhydrid in der Essigsäure). Beim Abkühlen setzte die Kristallisation unterhalb von 60 °C ein. Die Temperatur wurde im Laufe von 6 Stunden weiter auf 20 °C gesenkt. Die erhaltene Kristallsuspension wurde über eine Nutsche abgesaugt, dreimal mit je 25 ml einer Mischung aus 90 % Essigsäure und 10 % Acetanhydrid gewaschen und der Filterkuchen bei 60 °C/65 mbar im leichten Luftstrom getrocknet. Ausbeute: 179,2 g (79,6 % d.Th.) 12F-Dianhydrid, Schmelzverhalten: Phasenumwandlung: 115-120 °C unter Schmelzen und erneutem Erstarren, Schmelzpunkt: 168-170 °C, Anhydridgruppengehalt nach Titration mit n/10 Natronlauge/ n/10 Salzsäure: 2,625 mVal COOH/g (ber. 2,625).

| Analyse für $C_{34}H_{14}F_{12}O_7$: | | | |
|---|---|---|---|
| ber.: | C 53,54 % | H 1,84 % | F 29,92 % |
| gef.: | C 53,40 % | H 2,00 % | F 29,30 % |

**Patentansprüche**

**1.** Verbindungen der Formel

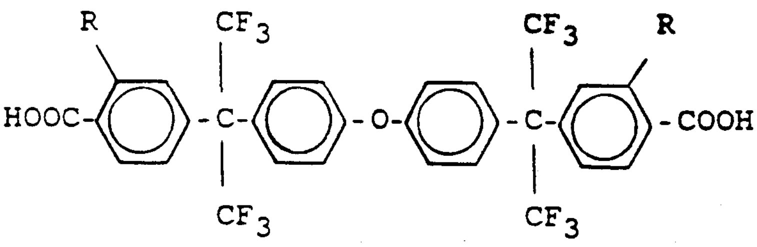

(I)

in der R gleich ist und Wasserstoff oder -COOH darstellt, sowie das Säurechlorid mit der Maßgabe, daß R Wasserstoff ist, und das Dianhydrid mit der Maßgabe, daß R den Rest -COOH darstellt.

**2.** Verfahren zur Herstellung

a) einer Verbindung der Formel

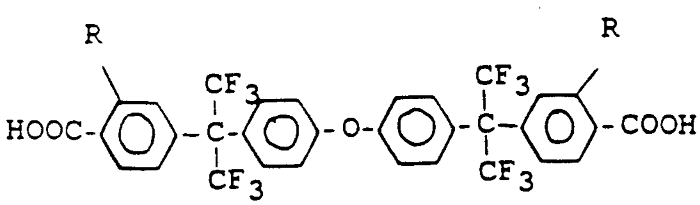

(II)

in der R gleich ist und Wasserstoff oder -COOH darstellt, oder

b) deren Anhydrid, wenn R nicht Wasserstoff ist, oder

c) das Säurechlorid, wenn R Wasserstoff ist, durch Luftoxidation in saurem Medium bei erhöhtem Druck und erhöhter Temperatur in Gegenwart eines Katalysatorgemischs, dadurch gekennzeichnet, daß die entsprechende Methylverbindung in einem sauren organischen Medium durch Einleiten von Luftsauerstoff bei Temperaturen von 120 bis 220 °C und einem Druck zwischen 5 und 40 bar, wobei an der Einleitstelle des Sauerstoffs der Sauerstoffpartialdruck mindestens 1 bar beträgt, in Gegenwart von mindestens zwei Schwermetallverbindungen sowie von Bromidionen oxidiert und als solche isoliert wird oder das erhaltene Reaktionsgut in das Dianhydrid b) oder in das Säurechlorid c) der Verbindung der Formel (II) übergeführt wird.

**3.** Verfahren nach Anspruch 2, dadurch gekennzeichnet, daß Essigsäure und/oder Propionsäure eingesetzt werden.

**4.** Verfahren nach Anspruch 2 oder 3, dadurch gekennzeichnet, daß die Reaktionstemperatur 140 bis 190, insbesondere 155 bis 180 °C beträgt.

**5.** Verfahren nach einem oder mehreren der Ansprüche 2 bis 4, dadurch gekennzeichnet, daß bei einem Druck zwischen 10 und 30, insbesondere zwischen 14 und 20 bar gearbeitet wird.

**6.** Verfahren nach einem oder mehreren der Ansprüche 2 bis 5, dadurch gekennzeichnet, daß der zur Oxidation eingesetzte Luftsauerstoff einen Sauerstoffgehalt von über 21 Vol-% besitzt und daß an der Einleitstelle des Sauerstoffs der Sauerstoffpartialdruck vorzugsweise 2 bis 15, insbesondere 3 bis 10 bar beträgt.

**7.** Verfahren nach einem oder mehreren der Ansprüche 2 bis 6, dadurch gekennzeichnet, daß der Sauerstoffpartialdruck in der Gasphase über dem Reaktionsmedium, der sich aus der Formel

$$P_{O_2} = \text{Volumen-\% } O_2\ (P_{gesamt} - P_{Essigsäuredampfdruck})$$

ergibt, mindestens 0,2 bar, vorzugsweise 0,35 bis 2,8, insbesondere 0,45 bis 1,3 bar beträgt.

**8.** Verfahren nach einem oder mehreren der Ansprüche 2 bis 7, dadurch gekennzeichnet, daß als Schwermetallionen Kobalt, Mangan und/oder Cer eingesetzt und diese in Form von Acetatverbindungen zugesetzt werden.

**9.** Verfahren nach einem oder mehreren der Ansprüche 2 bis 7, dadurch gekennzeichnet, daß Brom in Form von Bromiden oder als Lösung von Bromwasserstoff in Wasser oder Eisessig eingesetzt wird.

**10.** Verfahren nach einem oder mehreren der Ansprüche 2 bis 9, dadurch gekennzeichnet, daß das Mol-Verhältnis von Kobalt zu Mangan gleich 3 : 1 bis 1 : 3 ist, wobei die Summe der Konzentrationen der beiden Elemente Kobalt und Mangan gleich 0,01 bis 0,20 Grammatom/kg Gesamtreaktionsmasse, vorzugsweise 0,02 bis 0,12, insbesondere 0,04 bis 0,08 Grammatom/kg beträgt.

**11.** Verfahren nach einem oder mehreren der Ansprüche 2 bis 10, dadurch gekennzeichnet, daß das Mol-Verhältnis der Summe von Kobalt und Mangan zu Brom 1 : (0,01 bis 0,8), vorzugsweise 1 : (0,05 bis 0,4) oder, wenn in Formel (II) R Wasserstoff ist, das Molverhältnis 1 : (0,01 bis 2), vorzugsweise 1 : (0,025 bis 1), insbesondere 1 : (0,05 bis 0,2) beträgt.

**12.** Verfahren nach einem oder mehreren der Ansprüche 2 bis 10, dadurch gekennzeichnet, daß als zusätzliches Metallion Cer im Katalysator enthalten ist im Mol-Verhältnis von der Summe von Kobalt und Mangan zu Cer wie 1 : (0,02 bis 1,2), vorzugsweise 1 : (0,05 bis 0,6) oder, wenn R Wasserstoff ist, im Molverhältnis wie 1 : (0,02 bis 2), vorzugsweise 1 : (0,05 bis 1), insbesondere 1 : (0,2 bis 0,6).

**13.** Verfahren nach einem oder mehreren der Ansprüche 2 bis 11, dadurch gekennzeichnet, daß das Molverhältnis von Kobalt zu Cer 1 : (0,02 bis 1,2) beträgt, wenn in Formel (II) R die Gruppe -COOH ist.

**14.** Verfahren nach einem oder mehreren der Ansprüche 2 bis 13, dadurch gekennzeichnet, daß der Reaktionsansatz nach Beendigung der exothermen Reaktion für 1 bis 3 Stunden auf 150 bis 190 °C bei

einem Sauerstoffpartialdruck von 0,4 bis 2 bar gehalten wird, wenn in Formel (II) R die Gruppe -COOH ist.

**15.** Verfahren nach einem oder mehreren der Ansprüche 2 bis 14, dadurch gekennzeichnet, daß die Reaktion bei einer Wasserkonzentration der Monocarbonsäure von 2 bis 12, vorzugsweise 2 bis 7 und insbesondere bei 3 bis 5 % durchgeführt wird, wenn in Formel (II) R die Gruppe -COOH ist.

**16.** Verfahren nach einem oder mehreren der Ansprüche 2 bis 15, dadurch gekennzeichnet, daß das Dianhydrid unter Verwendung von Acetanhydrid in geringem Überschuß gebildet wird.

**17.** Verfahren nach einem oder mehreren der Ansprüche 2 bis 16, dadurch gekennzeichnet, daß das Anhydrid durch destillative Entfernung von Wasser aus der Reaktionslösung bei einer Temperatur oberhalb von 140 °C, gegebenenfalls unter Druck, gebildet wird.

**18.** Verfahren nach Anspruch 17, dadurch gekennzeichnet, daß zur Vervollständigung der Umsetzung Acetanhydrid zugesetzt wird bis zu einem Gehalt von 3 bis 12 % in der Lösung.

**19.** Verfahren nach einem oder mehreren der Ansprüche 2 bis 15, dadurch gekennzeichnet, daß das Dianhydrid durch Erhitzen der Tetracarbonsäure auf 180 bis 190 °C unter vermindertem Druck hergestellt wird.

**20.** Verfahren nach einem oder mehreren der Ansprüche 2 bis 15, dadurch gekennzeichnet, daß das Dianhydrid durch destillative Entfernung des Wassers aus der wasserfeuchten Tetracarbonsäure, die in einem mit Wasser nicht mischbaren Lösungsmittel suspendiert ist, gebildet wird.

**21.** Verfahren nach Anspruch 20, dadurch gekennzeichnet, daß als Lösungsmittel Toluol, o-Xylol, Tetrahydronaphthalin, Acetophenon oder Diphenylether eingesetzt wird.

**22.** Verwendung der Verbindungen gemäß Anspruch 1, in denen R = COOH ist, zur Herstellung von Polykondensaten, insbesondere von Polyimiden, Polycarbonsäureamiden, Polyamidcarbonsäureestern, Polyamiden und Imidoligomeren.

**23.** Verwendung der Verbindungen gemäß Anspruch 1, in denen R Wasserstoff ist, zur Herstellung von linearen Polycarbonsäureamiden und -carbonsäureestern, insbesondere Formkörpern, Filmen und Fasern daraus.

## Claims

**1.** A compound of the formula

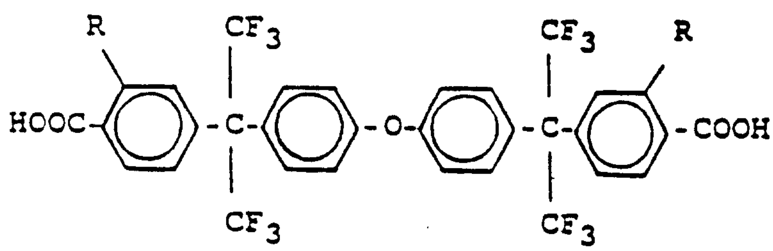

(I)

in which the R radicals are identical and are hydrogen or -COOH, or its acid chloride, in which case R is hydrogen, or its dianhydride, in which case R is the radical -COOH.

**2.** A process for the preparation

a) of a compound of the formula

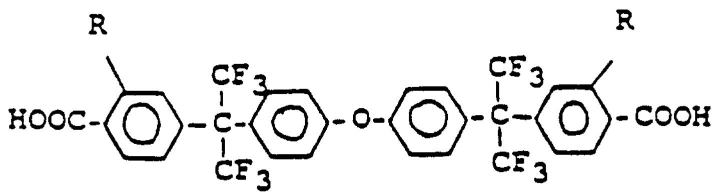

(II)

in which the Rs are identical and represent hydrogen or -COOH, or
b) the anhydride thereof, if R is not hydrogen, or
c) the acid chloride, if R is hydrogen, by atmospheric oxidation in an acid medium under increased pressure and at elevated temperature in the presence of a catalyst mixture, which process comprises oxidizing the corresponding methyl compound in an acid organic medium by passing in atmospheric oxygen at temperatures of 120 to 220 ° C and under a pressure of between 5 and 40 bar, the oxygen partial pressure at the oxygen inlet point being at least 1 bar, in the presence of at least two heavy metal compounds and of bromide ions, and isolating the product as such or converting the resulting reaction product into the dianhydride b) or into the acid chloride c) of the compound of the formula (II).

**3.** The process as claimed in claim 2, wherein acetic acid and/or propionic acid are used.

**4.** The process as claimed in claim 2 or 3, wherein the reaction temperature is 140 to 190, in particular 155 to 180 ° .

**5.** The process as claimed in one or more of claims 2 to 4, wherein a pressure of between 10 and 30, in particular between 14 and 20 bar is employed.

**6.** The process as claimed in one or more of claims 2 to 5, wherein the atmospheric oxygen used for the oxidation has an oxygen content of more than 21% by volume and wherein the oxygen partial pressure at the oxygen inlet point is preferably 2 to 15 and in particular 3 to 10 bar.

**7.** The process as claimed in one or more of claims 2 to 6, wherein the oxygen partial pressure in the gas phase above the reaction medium, which is given by the formula

$$P_{O_2} = \% \text{ by volume of } O_2 \ (P_{total} - P_{acetic\ acid\ vapor\ pressure}),$$

is at least 0.2 bar, preferably 0.35 to 2.8 and in particular 0.45 to 1.3 bar.

**8.** The process as claimed in one or more of claims 2 to 7, wherein cobalt, manganese and/or cerium are used as heavy metal ions and these are added in the form of acetate compounds.

**9.** The process as claimed in one or more of claims 2 to 7, wherein bromine is used in the form of bromides or as a solution of hydrogen bromide in water or glacial acetic acid.

**10.** The process as claimed in one or more of claims 2 to 9, wherein the molar ratio of cobalt to manganese is equal to 3:1 to 1:3, the sum of the concentrations of the two elements cobalt and manganese being equal to 0.01 to 0.20 gram atom/kg total reaction product, preferably 0.02 to 0.12 and in particular 0.04 to 0.08 gram atom/kg.

**11.** The process as claimed in one or more of claims 2 to 10, wherein the molar ratio of the sum of cobalt and manganese to bromine is 1:(0.01 to 0.8), preferably 1:(0.05 to 0.4), or, if R is hydrogen in formula (II), the molar ratio is 1:(0.01 to 2), preferably 1:(0.025 to 1) and in particular 1:(0.05 to 0.2).

**12.** The process as claimed in one or more of claims 2 to 10, wherein the catalyst contains, as an additional metal ion, cerium in a molar ratio of the sum of cobalt and manganese to cerium such as 1:-

(0.2 to 1.2), preferably 1:(0.05 to 0.6) or, if R is hydrogen, in a molar ratio such as 1:(0.02 to 2), preferably 1:(0.05 to 1) and in particular 1:(0.2 to 0.6).

**13.** The process as claimed in one or more of claims 2 to 11, wherein the molar ratio of cobalt to cerium is 1:(0.02 to 1.2) if R is the -COOH group in formula (II).

**14.** The process as claimed in one or more of claims 2 to 13, wherein the reaction batch is kept at 150 to 190 °C under an oxygen partial pressure of 0.4 to 2 bar for 1 to 3 hours after the exothermic reaction has ended, if R is the -COOH group in formula (II).

**15.** The process as claimed in one or more of claims 2 to 14, wherein the reaction is carried out at a water concentration of the monocarboxylic acid of 2 to 12, preferably 2 to 7 and in particular 3 to 5%, if R is the -COOH group in formula (II).

**16.** The process as claimed in one or more of claims 2 to 15, wherein the dianhydride is formed using a small excess of acetic anhydride.

**17.** The process as claimed in one or more of claims 2 to 16, wherein the anhydride is formed by removal of water from the reaction solution by distillation at a temperature above 140 °C, if appropriate under pressure.

**18.** The process as claimed in claim 17, wherein acetic anhydride is added up to a content of 3 to 12% in the solution in order to bring the reaction to completion.

**19.** The process as claimed in one or more of claims 2 to 15, wherein the dianhydride is prepared by heating the tetracarboxylic acid at 180 to 190 °C under reduced pressure.

**20.** The process as claimed in one or more of claims 2 to 15, wherein the dianhydride is formed by removal of the water by distillation from the water-moist tetracarboxylic acid which is suspended in a water-immiscible solvent.

**21.** The process as claimed in claim 20, wherein toluene, o-xylene, tetrahydronaphthalene, acetophenone or diphenyl ether is used as the solvent.

**22.** The use of a compound as claimed in claim 1, in which R is COOH, for the preparation of polycondensates, in particular polyimides, polycarboxylic acid amides, polyamidocarboxylic acid esters, polyamides and imide oligomers.

**23.** The use of a compound as claimed in claim 1, in which R is hydrogen, for the preparation of linear polycarboxylic acid amides and polycarboxylic acid esters, in particular molded articles, films and fibers therefrom.

**Revendications**

**1.** Composés de formule I ci-dessous

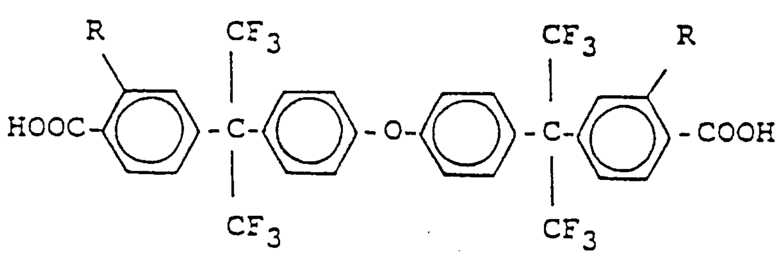

(I)

dans laquelle les deux R sont identiques et chacun l'hydrogène ou le groupe -COOH, ainsi que le chlorure d'acide avec la condition que R soit l'hydrogène, et le dianhydride avec la condition que R soit

le groupe -COOH.

**2.** Procédé de préparation
a) d'un composé de formule

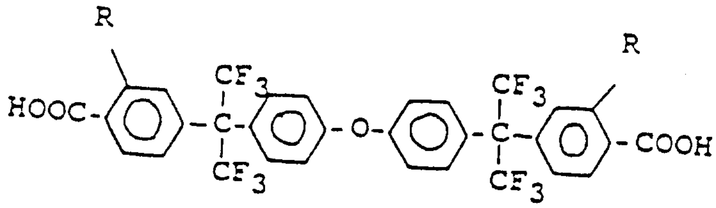

(II)

dans laquelle les deux R sont identiques et chacun l'hydrogène ou le groupe -COOH, ou bien
b) de son anhydride si R n'est pas l'hydrogène ou
c) du chlorure d'acide si R est l'hydrogène, par oxydation à l'air en milieu acide, sous pression et à chaud et en présence d'un mélange catalytique, procédé caractérisé en ce que l'on oxyde le composé méthylique correspondant dans un milieu organique acide en y faisant arriver un courant d'oxygène d'air à des températures de 120 à 220 °C et sous une pression de 5 à 40 bar, la pression partielle d'oxygène étant d'au moins 1 bar au lieu d'arrivée de l'oxygène, en présence d'au moins deux composés de métaux lourds ainsi que d'ions bromure, et on isole tel quel le produit formé ou bien on le transforme en le dianhydride b) ou le chlorure d'acide c).

**3.** Procédé selon la revendication 2, caractérisé en ce que l'on opère dans un milieu d'acide acétique et/ou d'acide propionique.

**4.** Procédé selon la revendication 2 ou 3, caractérisé en ce que la température de réaction est comprise entre 140 et 190 °C, en particulier entre 155 et 180 °C.

**5.** Procédé selon une ou plusieurs des revendications 2 à 4, caractérisé en ce que l'on opère sous une pression de 10 à 30 bar, en particulier de 14 à 20 bar.

**6.** Procédé selon une ou plusieurs des revendications 2 à 5, caractérisé en ce que l'air servant à l'oxydation a une teneur en oxygène de plus de 21 % en volume et en ce que au lieu d'arrivée de l'oxygène la pression partielle d'oxygène est de préférence de 2 à 15 bar, notamment de 3 à 10 bar.

**7.** Procédé selon une ou plusieurs des revendications 2 à 6, caractérisé en ce que la pression partielle d'oxygène dans la phase gazeuse surmontant le milieu de réaction, qui se déduit de la formule

$$P_{O_2} = \text{volume-\% } O_2 \; (P_{\text{totale}} - P_{\text{pression de vapeur de l'acide acétique}}),$$

est d'au moins 0,2 bar, de préférence de 0,35 à 2,8 bar et plus particulièrement de 0,45 à 1,3 bar.

**8.** Procédé selon une ou plusieurs des revendications 2 à 7, caractérisé en ce que les ions de métaux lourds sont des ions de cobalt, de manganèse et/ou de cérium, métaux qui sont ajoutés à l'état d'acétates.

**9.** Procédé selon une ou plusieurs des revendications 2 à 7, caractérisé en ce que le brome est à l'état de bromures ou d'une solution de bromure d'hydrogène dans de l'eau ou de l'acide acétique cristallisable.

**10.** Procédé selon une ou plusieurs des revendications 2 à 9, caractérisé en ce que le rapport molaire du cobalt au manganèse est compris entre 3:1 et 1:3, la somme des concentrations des deux éléments cobalt et manganèse étant de 0,01 à 0,20 atome-gramme par kg de la masse réactionnelle totale, de préférence de 0,02 à 0,12 et en particulier de 0,04 à 0,08 atome-gramme/kg.

**11.** Procédé selon une ou plusieurs des revendications 2 à 10, caractérisé en ce que le rapport molaire de la somme du cobalt et du manganèse au brome est de 1:(0,01 à 0,8), de préférence de 1:(0,05 à 0,4) ou bien, si dans le composé (II) R est l'hydrogène, ce rapport molaire est de 1:(0,01 à 2), de préférence de 1:(0,025 à 1) et notamment de 1:(0,05 à 0,2).

**12.** Procédé selon une ou plusieurs des revendications 2 à 10, caractérisé en ce que le catalyseur contient également du cérium comme ion de métal supplémentaire, dans un rapport molaire de la somme du cobalt et du manganèse au cérium tel que 1:(0,02 à 1,2), de préférence de 1:(0,05 à 0,6) ou bien, si R est l'hydrogène, dans le rapport molaire de 1:(0,02 à 2), de préférence de 1:(0,05 à 1) et notamment de 1:(0,2 à 0,6).

**13.** Procédé selon une ou plusieurs des revendications 2 à 11, caractérisé en ce que le rapport molaire du cobalt au cérium est de 1:(0,02 à 1,2) si dans le composé (II) R est le groupe -COOH.

**14.** Procédé selon une ou plusieurs des revendications 2 à 13, caractérisé en ce que si dans le composé (II) R est le groupe -COOH, quand la réaction exothermique est terminée on maintient le mélange de réaction pendant 1 à 3 heures entre 150 et 190 °C sous une pression partielle d'oxygène de 0,4 à 2 bars.

**15.** Procédé selon une ou plusieurs des revendications 2 à 14, caractérisé en ce que si dans le composé (II) R est le groupe -COOH, on effectue la réaction à une teneur en eau de l'acide monocarboxylique de 2 à 12 %, de préférence de 2 à 7 % et plus spécialement de 3 à 5 %.

**16.** Procédé selon une ou plusieurs des revendications 2 à 15, caractérisé en ce que l'on forme le dianhydride en présence d'un léger excès d'anhydride acétique.

**17.** Procédé selon une ou plusieurs des revendications 2 à 16, caractérisé en ce que l'on forme l'anhydride en éliminant l'eau par distillation de la solution réactionnelle à une température supérieure à 140 °C, éventuellement sous pression.

**18.** Procédé selon la revendication 17, caractérisé en ce que pour compléter la réaction on ajoute de l'anhydride acétique jusqu'à une teneur de la solution de 3 à 12 %.

**19.** Procédé selon une ou plusieurs des revendications 2 à 15, caractérisé en ce que l'on forme le dianhydride en chauffant l'acide tétracarboxylique entre 180 et 190 °C sous pression réduite.

**20.** Procédé selon une ou plusieurs des revendications 2 à 15, caractérisé en ce que l'on forme le dianhydride en éliminant par distillation l'eau de l'acide tétracarboxylique humide d'eau en suspension dans un solvant non miscible à l'eau.

**21.** Procédé selon la revendication 20, caractérisé en ce que l'on emploie comme solvant du toluène, du o-xylène, du tétrahydronaphtalène, de l'acétophénone ou de l'éther diphénylique.

**22.** Emploi des composés de la revendication 1 dans lesquels R est le groupe -COOH pour fabriquer des produits de polycondensation, en particulier polyimides, polycarboxylamides, esters d'acides polyamidocarboxyliques, polyamides et imides oligomères.

**23.** Emploi des composés de la revendication 1 dans lesquels R est l'hydrogène pour fabriquer des polycarboxylamides et esters linéaires, en particulier des objets moulés, pellicules et fibres de ces matières.